Europäisches Patentamt

European Patent Office

Office européen des brevets

(19)

(11) Veröffentlichungsnummer: **0 239 086 B1**

# EUROPÄISCHE PATENTSCHRIFT

(12)

(45) Veröffentlichungstag der Patentschrift: **03.06.92**

(21) Anmeldenummer: **87104374.1**

(22) Anmeldetag: **25.03.87**

(51) Int. Cl.⁵: **C09B 67/42**, C09B 61/00, A23L 1/275

(54) **Verfahren zur Herstellung von feinteiligen, wasserdispergierbaren Carotinoid-Präparationen.**

(30) Priorität: **26.03.86 DE 3610191**

(43) Veröffentlichungstag der Anmeldung:
**30.09.87 Patentblatt 87/40**

(45) Bekanntmachung des Hinweises auf die
Patenterteilung:
**03.06.92 Patentblatt 92/23**

(84) Benannte Vertragsstaaten:
**BE CH DE FR GB IT LI NL**

(56) Entgegenhaltungen:
**DE-A- 2 053 381**
**DE-B- 1 190 314**
**FR-A- 2 030 364**
**FR-A- 2 414 530**

(73) Patentinhaber: **BASF Aktiengesellschaft**
**Carl-Bosch-Strasse 38**
**W-6700 Ludwigshafen(DE)**

(72) Erfinder: **Lueddecke, Erik, Dr.**
**Thomas-Mann-Strasse 27**
**W-6704 Mutterstadt(DE)**
Erfinder: **Horn, Dieter, Dr.**
**Schroederstrasse 69**
**W-6900 Heidelberg(DE)**

## Beschreibung

Die Erfindung betrifft ein Verfahren zur Herstellung von feinteiligen und farbstarken Carotinoid-Emulsionen und den daraus gewonnenen Carotinoid-haltigen, kaltwasserdispergierbaren Trockenpulvern unter Verwendung einer Mischung aus des Ester einer langkettigen Fettsäure mit Ascorbinsäure und einem kaltwasserlöslichen Stärkeprodukt als Kolloid.

Die Farben einer Vielzahl von Pflanzen und Tieren werden durch Substanzen bestimmt, die man der Gruppe der Carotinoide zurechnet. Es sind dies Verbindungen, die eine intensive gelbe bis rote Farbe haben. Neben den in der Natur vorkommenden Carotinoiden sind auch solche bekannt, die nur synthetisch hergestellt werden können. Wichtigste Vertreter beider Klassen sind ß-Carotin, Canthaxantin, Citranaxanthin, ß-Apo-8'carotinal sowie Ester der ß-Apo-8'-carontinsäure. Bedingt durch ihre gesundheitliche Unbedenklichkeit bzw. durch die z.T. sogar vorhandenen gesundheitsfördernde Pro-Vitamin-A-Wirkung werden diese Verbindungen in steigendem Maße als Farbstoffe bzw. Zusätze für Lebensmittel, Pharmazeutika, kosmetika sowie Futtermittel eingesetzt.

Während die Anfärbung von öligen bzw. fettigen Systemen mit den reinen kristallinen Substanzen einigermaßen gut möglich ist, können Jedoch wäßrige Systeme damit praktisch nicht gefärbt werden. Auch die Verwertung von der Nahrung bzw. dem Futter beigemischten reinen Carotinoiden durch den menschlichen bzw. tierischen Organismus ist sehr schlecht. Die Ursache hierfür liegt in der völligen Unlöslichkeit der meisten Carotinoide in Wasser. Selbst in organischen Lösungsmitteln wie Alkoholen und Alkanen ist die Löslichkeit der Carotinoide nur sehr begrenzt.

Gemäß der US-Patentschrift 2,861,891 wurde daher bereits ein Verfahren zur Herstellung von wasserdispergierbaren Carotinpräparationen beschrieben. Danach wird das Carotinoid, z.B´ ß-Carotin als einer der wichtigsten Vertreter dieser Gruppe, in einem Pflanzenöl bei erhöhter Temperatur gelöst, diese Öl-Lösung in einem wäßrigen Schutzkolloid-System emulgiert und die resultierende Emulsion durch Wasserentzug in ein Trockenpulver überführt.

Wird das Trockenpulver in warmem Wasser redispergiert, so bildet sich erneut eine trübe, orangegelbe Emulsion. Diese kann z.B zum Färben von Lebensmitteln eingesetzt werden. Wichtige anwendungstechnische Eigenschaften dieser Färbepräparate sind Löslichkeit, Farbton, Farbstärke` Trübung und Stabilität im Anwendungsmedium. Von Vorteil für viele Anwender ist dabei eine gute Löslichkeit in kaltem Wasser, ein intensiver gelber Farbton, eine niedrige Trübung und eine hohe Stabilität auch in saurer Lösung (2<ph<4).

Nach dem Stand der Technik werden diese Eigenschaften teilweise erreicht durch Verwendung von Gummi-arabicum als Schutzkolloid in der wäßrigen Phase. Der Einsatz von Gummi-arabicum in einem Lebensmittelzusatzstoff ist Jedoch in jüngster Zeit sehr problematisch geworden, da die Qualitäten sehr stark schwanken und darüber hinaus auch der mikrobiologische Status dieses vom Menschen gesammelten Naturproduktes nicht den Erfordernissen einer qualitätsbewußten Lebensmittelproduktion entspricht.

Gemäß H. Kläui in wiss. Veröffentl. Deutsch. Ges. f. Ernährung 9, (1963) S. 396 ist auch schon Dextrin als Schutzkolloid genannt. Dort wird jedoch darauf hingewiesen, daß die erhaltenen Produkte wegen ihrer Tendenz zum Aufrahmen in Citrusgetränken noch nicht voll befriedigen.

Es bestand daher die Aufgabe ein Schutzkolloid vorzuschlagen, das die genannten Nachteile nicht aufweist und das dem Carotinoidpräparat sämtliche geforderten Eigenschaften verleiht.

Diese Aufgabe wurde erfindungsgemäß gelöst durch ein Verfahren zur Herstellung von wasserdispergierbaren Carotinoid-Zubereitungen durch Auflösen des Carotinoids in einem Trägeröl bei erhöhter Temperatur bis zur Sättigung, schnelles Emulgieren mit einem wäßrigen Schutzkolloid und anschließendem Entfernen des Wassers, wobei als Schutzkolloid eine Mischung aus dem Ester einer langkettigen Fettsäure mit Ascorbinsäure und einem kaltwasserlöslichen Stärkeprodukt verwendet wird.

Dieses Verfahren erlaubt die Herstellung einer Gummi-arabicum-freien Carotinoidpräparation, die in getrocknetem Zustand eine sehr gute Kaltwasserdispergierbarkeit aufweist, im dispergierten Zustand bei hoher spezifischer Farbstärke und niedriger Trübung einen intensiv gelben Farbton zeigte und sowohl im getrockneten als auch im dispergierten Zustand eine hohe Stabilität hat

Im einzelnen wendet man zweckmäßig folgende Mischungen an:

1. Als Schutzkolloidlösung wird ein Gemisch aus einem kaltwasserlöslichen Stärkeprodukt, Ascorbylpalmitat, gegebenenfalls zusätzlich Lecithin, Zucker, in Wasser eingesetzt. Aus der Vielzahl der kommerziell verfügbaren kaltwasserlöslichen Stärkeprodukte haben nur bestimmte eine ausreichende Emulgieraktivität. Die Auswahl kann anhand der erzielten Teilchengröße der Emulsionströpfchen einer nach den Angaben des Beispiel 1 (unter Variation des Dextrins) hergestellten Emulsion vorgenommen werden. Die Tröpfchengröße - bestimmt mit der photonen-Korrelations-Spektroskopie - muß einen mittleren Durchmesser von 0,5 μm unterschreiten. Die Menge Dextrin, bezogen auf das Endprodukt, liegt zwischen 5 und 90 Go.-%, bevorzugt zwischen 10 und 50 Cew.-%, ganz besonders bevorzugt bei 15 bis 30 Gew.-%. Als kaltwasserlösliche Stärkeprodukte kommen insbesondere Dextrin oder modifizierte Stärke z.B. Stärkeoctenylsuccinat in Betracht.

Dextrin ist eine teilweise hydrolgsierte Stärke, die entweder durch wärme allein oder durch wärme in Gegenwert von Säuren aus verschiedenen Stärken (Mais-, Kartoffel-, Weizen-, Beisstärke etc.) gewonnen wird, wie dies z.B. in "Food Chemical Codex", Third Edition 1981, National Academy Press, S. 96 oder in "Starch" Chemistry and Technology Academie Press, Inc. (1984) Second Edition beschrieben ist. Der Hydrolysegrad wird dabei im allgemeinen als Dextrose Equivalent (DE) angegeben. Er ist ein Maß für den Anteil an reduzierenden Zucker berechnet als D-Glucose auf Trockengewicht-Basis. Der DE-Wert steht in umgekehrter Beziehung zum Polymerisationsgrad. Unhydrolysierte Stärke hat einen DE-Wert von praktisch 0, während wasserfreie D-Glucose definitionsgemäß den DE-Wert 100 hat.

In Betracht kommendes Dextrin hat beispielsweise einen DE-Wert von weniger als 20 vorzugsweise 1 bis 8.

Das Verhältnis des kaltwasserlöslichen Stärkeprodukts zum Ester einer langkettigen Fettsäure mit Ascorbinsäure beträgt im allgemeinen 100:1 bis 1:1 vorzugsweise 50:1 bis 10:1.

Der Ester einer langkettigen Fettsäure, das ist in der Regel eine Fettsäure mit z.B. 18 Kohlenstoffatomen, mit Ascorbinsäure, insbesondere Ascorbylpalmitat (AP), hat in dieser Schutzkolloidlösung eine Doppelfunktion: Zum einen wirkt er als Emulgator, d. h. die Emulsion wird erst durch Zusatz des AP aufrahmstabil und die erreichbare Tröpfchengröße wird wunschgemäß verringert. Diese Wirkung war überraschend, da in der Literatur die Emulgatorwirkung des AP nur dem entsprechenden Natriumsalz zugeschrieben wird (DE-PS 1 190 314); in der vorliegenden Schutzkolloidlösung liegt der pH-Wert Jedoch im sauren pH, so daß AP nicht als Salz vorliegt. In zweiter Linie wirkt es - wie literaturbekannt - als Antioxidans. Die Konzentration liegt zwischen 0,5 und 4 %, bevorzugt bei 2 Gew.-%, bezogen auf das fertige Trockenpulver.

Ein Lecithin-Zusatz erhöht zusätzlich die Stabilität des Carotinoids gegen Hitze- und Lichteinflüsse, die Konzentration liegt zwischen 0 und 2 Gew.-%, bevorzugt bei 1 Gew.-%.

Ein Anteil Zucker in der Rezeptur dient als Weichmacher und erhöht die mechanische und thermische Stabilität des Endproduktes. Bevorzugt wird Glucosesirup eingesetzt; die Menge hängt vom Anteil des kaltwasserlöslichen Stärkeprodukts ab und liegt zwischen 0 und 85 %, bevorzugt bei 65 %.

Der Anteil Wasser wird so gewählt, daß eine niederviskose Emulsion resultiert, die Viskosität sollte bei 60° C zwischen 5 und 50 mPas liegen, bevorzugt wird 10 mPas.

2. Das Carotinoid wird in Pflanzenöl dispergiert, fein gemahlen und mit Tocopherol als zusätzlichem Antioxidans versetzt. Als Pflanzenöl kann beispielsweise Orangenöl, Sojaöl, Baurnwollsamenöl oder Erdnußöl eingesetzt werden. Die Konzentration des Carotinoids in Öl sollte zwischen 10 und 30 Gew.-%, bevorzugt 20 Gew.-%, betragen; die Mahlung des Carotinoids sollte zu einer Partikelgrößenverteilung führen, die Kristalle > 10 μm weitgehend ausschließt. Dadurch wird eine gleichmäßige Lösungskinetik gewährleistet. Als Tocopherol kann sowohl natürliches als auch synthetisches Tocopherol (d-1-∝ -Tocopherol) eingesetzt werden. Das Mischverhältnis Carotinoid zu Tocopherol liegt bei 3 : 1 bis 1 3, bevorzugt 1 1. Die resultierende Dispersion wird nachfolgend als "Ölphase" bezeichnet.

Die Öphase wird nun kurzzeitig erhitzt bis sich das Carotinoid im Trägeröl vollständig auflöst. Die erhaltene Carotin-Lösung wird in die wäßrige Schutzkolloid-Lösung eingeleitet und emulgiert. Die Menge der Öphase wird so bemessen, daß im Endprodukt die gewünschte Carotinoid-Konzentration erreicht wird. Diese liegt zwischen 0,1 und 2 %, optimal ist eine Konzentration von 1 %.

Die durch diese Maßnahmen erreichbare minimale Tröpfchengröße ist abhängig von der Art des Emulgierwerkzeuges, der Rezeptur und der Zeitdauer der Emulgierung. Bei Einsatz eines nach dem Prinzip des Rotor-Stator arbeitenden Dispergierverkzeugs und bei Wahl der bevorzugten Mengenverhältnisse der Einsatzstoffe wird eine mittlere Teilchengröße von 0,3 μm ± 50% erreicht. Dies ist für eine 0/W-Emulsion ungewöhnlich niedrig und im Hinblick auf das Endprodukt von großem Vorteil, da sowohl Farbstärke als auch Farbton und Trübung mit kleiner werdenden Tröpfchen in die genannte positive Richtung beeinflußt werden. Die Messung der Teilchengröße erfolgt wie bei der Auswahl der Dextrine mit der Methode der Photonen-Korrelations-Spektroskopie.

3. Die resultierende Emulsion ist lagerstabil und kann entweder - gegebenenfalls unter Zusatz von Konservierungsstoffen - direkt zum Färben von Lebensmitteln eingesetzt werden oder nach den bekannten Verfahren durch schonenden Wasserentzug, beispielsweise durch Sprühtrocknung, in ein Trockenpulver überführt werden, das die eingangs aufgeführten besonderen Eigenschaften hat.

Die eingangs geschilderten Eigenschaften der nach diesem Verfahren hergestellten Carotinoidpräparationen, nämlich gute Kaltwasserdispergierbarkeit, hohe spezifische Farbstärke, niedrige Trübung und - im Fall von $\beta$-Carotin - intensiv gelber Farbton, lassen sich in dieser Kombination mit anderen literaturbekannten Formulierverfahren nicht erreichen. So kann zwar z.B. nach DE-OS 25 34 091 durch Lösen des Carotinoids in einem flüchtigen organischen Lösungsmittel, Emulgieren dieser Lösung in einem wäßrigen Trägertoffsystem und anschließende Entfernung des organischen Lösungsmittels durch Verdampfen eine Dispersion mit sehr feinen Carotinoid-Teilchen und entsprechend niedriger Trübung hergestellt werden, die gemäß den beschriebenen Verfahren für die spezielle Färbung verantwortlichen Öltröpfchen mit molekular gelöstem $\beta$-Carotin sind nach diesem Verfahren jedoch nicht vorhanden. Weiterhin ist die nach DE-OS 25 34 091 notwendige Verwendung eines lebensmittelrechtlich nicht unbedenklichen Emulgators problematisch. Auch das in EP 65 193 beschriebenen Verfahren liefert partikuläre Carotinoid-Teilchen, die eine vom gelösten Carotin unterschiedliche Färbewirksamkeit haben.

Beispiel 1

In einem 1-l-Becherglas werden 375 ml Wasser, 75 g Dextrin (Nadex 4772 der Fa. National Starch), 253 g Glucosesirup (Glucodex 127 der Fa. Maizena), 6 g Ascorbylpalmitat und 1,65 g Lecithin (Emulfluid E der Fa. Lucas-Mayer) vorgelegt, homogen durchmischt und auf 60°C temperiert. Unabhängig davon werden in einem 250-ml-Vierhalsrundkolben 3 g DL-$\alpha$-Tocopherol und 18 g $\beta$-Carotin-Dispersion (20 %ige Dispersion in Erdnußöl) durchmischt. Der Rundkolben wird in einem auf 180°C aufgeheizten Ölbad erwärmt bis das $\beta$-Carotin vollständig in Lösung gegangen ist. Die Öl-Lösung wird dann unter Rühren mit einem Homogenisator (Ultra-Turax T 45 Fa. Janke und Kunkel) bei 10 000 U/Min in die Wasserphase eingeleitet. Nach einer Emulgierzeit von 15 Minuten liegt eine farbstarke, lagerstabile, gelb-orange Emulsion mit Öltröpfchen im Größenbereich von 0,3 $\mu$m vor. Nach Wasserentzug durch Sprühtrocknung erhält man ein kaltwasserdispergierbares, freifließendes Pulver mit einem $\beta$-Carotin-Gehalt von 1,1 %.

Vergleichsbeispiel 1

In einem 1-l-Becherglas werden 375 ml Wasser, 75 g Dextrin (Nadex 4772 der Fa. National Starch), 253 g Glucosesirup (Glucodex 127 der Fa.

Maizena) und 1,65 g Lecithin (Emulfluid E der Fa. Lucas Mayer) vorgelegt, homogen durchmischt und auf 60°C temperiert. Unabhängig davon werden in einem 250-ml-Vierhalsrundkolben 3 g DL-$\alpha$-Tocopherol und 18 g $\beta$-Carotin-Dispersion (20 %ige Dispersion in Erdnußöl) durchmischt. Der Rundkolben wird in einem auf 180°C aufgeheizten Ölbad erwärmt bis das $\beta$-Carotin vollständig in Lösung gegangen ist. Die Öl-Lösung wird dann unter Rühren mit einem Homogenisator (Ultra-Turrax T 45 Fa. Janke und Kunkel) bei 10 000 U/Min in die Wasserphase eingeleitet. Nach einer Emulgierzeit von 15 Minuten liegt eine trübe, orange Emulsion vor, die bei Lagerung bei 60°C innerhalb von 8 h leicht aufrahmt. Die Tröpfchengröße liegt bei 0,6 - 0,7 $\mu$m, die Größenverteilung ist sehr breit. Das durch Wiederauflösen des aus der Emulsion gewonnenen Trockenpulvers hergestellte Hydrosol ist ebenfalls trübe, leicht rotstichig und hat eine um ca. 30 % geringere spezifische Farbstärke als ein nach dem Beispiel 1 gewonnenes Hydrosol.

Beispiel 2

In einem 1-l-Becherglas werden 375 ml Wasser, 75 g Stärkeoctenylsuccinat (Capsul der Fa. National Starch), 253 g Glucosesirup (Glucodex 127 der Fa. Maizena), 6 g Ascorbylpalmitat und 1,65 g Lecithin (Emulfluid E der Fa. Lucas Mayer) vorgelegt, homogen durchmischt und auf 60°C temperiert. Unabhängig davon werden in einem 250 ml-Vierhalsrundkolben 3 g DL-$\alpha$-Tocopherol und 18 g $\beta$-Carotin-Dispersion (20 %ige Dispersion in Erdnußöl) durchmischt. Der Rundkolben wird in einem auf 180°C aufgeheizten Ölbad erwärmt bis das $\beta$-Carotin vollständig in Lösung gegangen ist. Die Öl-Lösung wird dann unter Rühren mit einem Homogenisator (Ultra-Turax T 45 Fa. Janke und Kunkel) bei 10 000 U/Min in die Wasserphase eingeleitet. Nach einer Emulgierzeit von 15 Minuten liegt eine sehr feine, farbstarke gelb-organge Emulsion mit Öltröpfchen im Größenbereich von 0,25 1m vor.

Vergleichsbeispiel 2

In einem 1-l-Becherglas werden 375 ml Wasser, 75 g Stärkeoctenylsuccinat (Capsul der Fa. National Starch), 253 g Glucosesirup (Glucodex 127 der Fa. Maizena) und 1,65 g Lecithin (Emulfluid E der Fa. Lucas Mayer) vorgelegt, homogen durchmischt und auf 60°C temperiert. Unabhängig davon werden in einem 250 ml-Vierhalsrundkolben 3 g DL-$\alpha$-Tocopherol und 18 g $\beta$-Carotin-Dispersion (20 %ige Dispersion in Erdnußöl) durchmischt. Der Rundkolben wird in einem auf 180°C aufgeheizten Ölbad erwärmt bis das $\beta$-Carotin vollständig in Lösung gegangen ist. Die Öl-Carotin vollständig in Lösung gegangen ist. Die Öl-Lösung wird dann unter Rühren mit einem Homogenisator (Ultra-Turrax T 45 Fa. Janke und Kunkel) bei 1O OOO U/Min in die Wasserphase eingeleitet. Nach einer Emulgierzeit von 15 Minuten liegt eine trübe, orange Emulsion vor, die bei Lagerung bei Raumtemperatur innerhalb von 15 h aufrahmt. Die Tröpfchengröße liegt bei O,5 1m, die Größenverteilung ist sehr breit.

## Patentansprüche

1. Verfahren zur Herstellung von wasserdispergierbaren Carotinoid-Zubereitungen durch Auflösen des Carotinoids in einem Trägeröl bei erhöhter Temperatur bis zur Sättigung, schnelles Emulgieren mit einem wäßrigen Schutzkolloid und anschließendes Entfernen des Wassers, dadurch gekennzeichnet, daß man als Schutzkolloid eine Mischung aus dem Ester einer langkettigen Fettsäure mit Ascorbinsäure und einem kaltwasserlöslichen Stärkeprodukt verwendet.

2. Verfahren gemäß Anspruch 1, dadurch gekennzeichnet, daß man ein Antioxydans-System aus Ascorbylpalmitat, Lecithin und Tocopherol verwendet.

3. Verfahren gemäß Anspruch 1, dadurch gekennzeichnet, daß man als kaltwasserlösliches Stärkeprodukt Dextrin oder eine chemische modifizierte Stärke verwendet.

4. Verfahren gemäß Anspruch 3, dadurch gekennzeichnet, daß man als chemisch modifizierte Stärke Stärkeoctenylsuccinat verwendet.

## Claims

1. A process for the preparation of a water-dispersible carotenoid formulation by dissolving the carotenoid in a carrier oil at elevated temperatures until saturation is achieved, rapidly emulsifying the solution with an agueous protective colloid and then removing the water, wherein the protective colloid used is a mixture of an ester of a long-chain fatty acid with ascorbic acid and a starch product which is soluble in cold water.

2. A process as claimed in claim 1, wherein an antioxidant system consisting of ascorbyl palmitate, lecithin and tocopherol is used.

3. A process as claimed in claim 1, wherein dextrin or chemically modified starch is used as the starch product which is soluble in cold water.

4. A process as claimed in claim 3, wherein starch octenyl succinate is used as the chemically modified starch.

## Revendications

1. Procédé d'obtention de préparations de carotinoïde dispersables dans l'eau par dissolution du carotinoïde à température élevée, dans un véhicule huileux jusqu'à saturation, émulsification rapide avec un protecteur colloïdal aqueux et élimination subséquente de l'eau, caractérisé par le fait que l'on utilise, comme protecteur colloïdal, un mélange de l'ester d'un acide gras à longue chaîne et d'acide ascorbique avec un produit amylacé soluble dans l'eau à froid.

2. Procédé selon la revendication 1, caractérisé par le fait que l'on utilise un système antioxydant composé de palmitate d'ascorbyle, de lécithine et de tocophérol.

3. Procédé selon la revendication 1, caractérisé par le fait que l'on utilise, comme produit amylacé soluble dans l'eau à froid, de la dextrine ou un amidon modifié chimiquement.

4. Procédé selon la revendication 3, caractérisé par le fait que l'on utilise, comme amidon modifié chimiquement, du succinate d'octényle amylacé.